Europäisches Patentamt

European Patent Office

Office européen des brevets

(19)

(11) Publication number: **0 045 032**
**B1**

(12) **EUROPEAN PATENT SPECIFICATION**

(45) Date of publication of patent specification: **21.03.84**

(21) Application number: **81105697.7**

(22) Date of filing: **20.07.81**

(51) Int. Cl.³: **C 12 Q 1/00, C 12 N 9/20, C 12 Q 1/44**

(54) **A triglyceride analysis composition and a method for triglyceride determination.**

(30) Priority: **22.07.80 US 171065**

(43) Date of publication of application:
**03.02.82 Bulletin 82/5**

(45) Publication of the grant of the patent:
**21.03.84 Bulletin 84/12**

(84) Designated Contracting States:
**BE DE FR GB IT LU NL SE**

(56) References cited:
**WO - A - 80/00260**
**DE - A - 2 323 609**
**US - A - 4 179 334**

**CHEMICAL ABSTRACTS, vol. 88, no. 5, 30th
January 1978, page 221, no. 34166r, Columbus,
Ohio, U.S.A. M. SUGIURA et al.: "Studies on
enzymes. Part CXLIV. A simple colorimetric
method for determination of serum triglycerides
with lipoprotein lipase and glycerol
dehydrogenase"**

(73) Proprietor: **Baker Instruments Corporation
2196 Avenue C. P.O. Box 2168
Bethlehem Pennsylvania 18001 (US)**

(72) Inventor: **Matteo, Martha R.
Greenmeadow Road
Pleasantville N.Y. 10570 (US)**
Inventor: **Battey, York C.
119-29 180 Street
St. Albans, N.Y. 11434 (US)**

(74) Representative: **Vossius Vossius Tauchner
Heunemann Rauh
Siebertstrasse 4 P.O. Box 86 07 67
D-8000 München 86 (DE)**

Courier Press, Leamington Spa, England.

A triglyceride analysis composition and a method for triglyceride determination

The invention relates to a composition and method for triglyceride determination and particularly for the triglyceride determination of animal serum, especially, human serum. In its broadest scope, the invention features a triglyceride analysis composition for use in the triglyceride determination of serum, consisting essentially of at least one bacterial lipase derived from a bacteria source selected from *Pseudomonas fluorescens* and/or *Chromobacterium viscosum* and in the amount of at least 700 lipase units per mg triglycerides in the serum, and a glycerol detection reagent.

Background of the invention

In many fields it is important to determine triglyceride concentration of a substance. This is particularly important in clinical biochemistry. The determination of triglyceride concentration in human serum is an important part of routine lipid analysis in clinical chemistry.

There are numerous prior art compositions and methods. The drawbacks of the prior art are primarily due to the time needed to carry out the methods, the accuracy and reliability of the determination, and the linearity of the determination for high levels of triglycerides concentration.

The instant invention overcomes these drawbacks by providing a fast and accurate endpoint reaction which does not require a pre-incubation step. By "endpoint reaction" it is meant that all of the triglycerides in a human serum can be converted to a detectable product. Typically, the determination can be made in less than 4 minutes from a single formulation or mixture of components. Furthermore, the invention is effective for a determination of serum triglycerides in a concentration of up to at least 1000 mg per dL of serum. Prior art methods require such high levels to be diluted to lower levels before an accurate measurement can be made.

DE—A2 323 609 refers to the determination of triglycerides using esterification by the action of lipase from *Pseudomonas* (viz. *Pseudomonas fluorescens*).

However, the use of bovine serum albumen and the specifically defined component ratios and ranges (e.g. 700 units lipase per milligram triglycerol) according to the present invention, which afford the linearity and speed of assay, are not disclosed.

A similar method is disclosed in WO-80/00260 (pages 7/8 — measure at 340 $\mu$m). The lipase is obtained from *Chromobacterium viscosum*. The glycerol detection reagent is different, however, relying on the formation of a colored formazan.

The process according to CA *88*: 34166r mentions *Pseudomonas fluorescens* lipase utilized in a serum triglyceride assay. However, the glycerol detection reagent system is different from the one in the present invention, the process being a chromogen one (measure at 370 $\mu$m).

According to the instant invention, determination of triglyceride in serum is accomplished by use of a triglyceride analysis composition consisting essentially of at least one bacterial lipase derived from a bacterial source selected from *Pseudomonas fluorescens* and/or *Chromobacterium viscosum* and in the amount of at least 700 lipase units per mg triglycerides in the serum and a glycerol detection reagent, said reagent consisting essentially of glycerol kinase, pyruvate kinase, lactate dehydrogenase, adenosine triphosphate, phosphoenol pyruvate, nicotinamide adenine dinucleotide (reduced form), bovine serum albumen, magnesium sulphate, and a buffer; the ratio of the pyruvate kinase to lactate dehydrogenase is about 1:1, the ratio of pyruvate kinase and lactate dehydrogenase each to glycerol kinase is about 2:1; the magnesium ions are present in the range from 3.1 to 6.0 mm; and the pH of the reagent is in the range of from 6.9 to 7.6.

The instant invention utilizes the surprising discovery that a bacterial lipase derived from a bacterial source selected from *Pseudomonas fluorescens* and/or *Chromobacterium viscosum* is effective for the complete and rapid hydrolysis of triglycerides in serum to glycerol. The lipase is combined with a glycerol detection reagent to define a composition for a so-called one-step triglyceride analysis.

In its broadest scope, the invention also features the method of triglyceride determination of a serum specimen, comprising the steps of combining the specimen with at least one bacterial lipase derived from a bacterial source selected from *Pseudomonas fluorescens* and/or *Chromobacterium viscosum* and in the amount of at least 700 lipase units per mg triglycerides in the specimen, and measuring the glycerol content obtained.

As used herein, "serum" refers to animal serum and particularly to human serum; and one "lipase unit" is defined as the amount which will release one micromole of fatty acid per minute from emulsified olive oil at 22°C and pH 7.5 in a reaction mixture as follows:

0.4 M sodium chloride
6 millimolar magnesium sulphate · $7H_2O$
5 millimolar calcium chloride · $2H_2O$
0.67 mg bovine serum albumen per milliliter of the reaction mixture
137 mg olive oil in suspension per milliliter of the reaction mixture

One preferred embodiment of the invention is a triglyceride analysis composition comprising a bacterial lipase derived from *Pseudomonas fluorescens*, and a glycerol detection reagent.

Another preferred embodiment of the invention features a triglyceride analysis composition consisting essentially of a bacterial lipase derived from *Chromobacterium viscosum*, and a glycerol detection reagent.

Yet another preferred embodiment features a triglyceride analysis composition consisting essentially of bacterial lipases derived from *Pseudomonas fluorescens* and *Chromobacterium viscosum*, and a glycerol detection reagent.

The use of at least 700 lipase units per mg triglycerides of serum is used. The number of lipase units used in a reaction mixture should be selected to hydrolyze the maximum amount of triglycerides present. For human serum, more than 1000 mg per dL of serum is unusual so that this can be used as a target level. Then, the results of the triglyceride determination would be reliable in that a person determined to have an above average triglycerides concentration would be correctly informed.

It is understood that the invention need not be limited to animal serum in view of known technology and the generalization is understood without being specifically pointed out.

As used herein, "reaction mixture" is a combination of the instant composition and the serum. The serum could be undiluted or diluted human or animal serum. Nevertheless, the serum will still be identified as "serum" or "animal serum" or "human serum" without specific reference to dilution.

In carrying out the invention, it is understood that the handling of the instant lipases is consistent with prior art practices as to storage, and environmental conditions for the reaction with the serum.

There are numerous prior art methods and compositions for measuring triglycerides concentration. One class of methods includes transforming the triglycerides into glycerol and then measuring the glycerol content. In addition, glycerol detection reagents are well known in the art, particularly for measurements using an automated analyzer.

Generally, a glycerol detection reagent is known in which the glycerol is phosphorylated with adenosine triphosphate in the presence of glycerol kinase to give glycerol-1-phosphate and adenosine diphosphate (see e.g. US—A—3,898,130; 3,862,009 and 4,066,508). The adenosine diphosphate is then converted by phosphoenol pyruvate in the presence of pyruvate kinase into pyruvate and adenosine triphosphate. The pyruvate formed is hydrogenated by nicotinamide adenine dinucleotide in the reduced form in the presence of lactate dehydrogenase to obtain lactate while the nicotinamide adenine dinucleotide is oxidized to nicotinamide adenine dinucleotide. The amount of nicotinamide adenine dinucleotide utilized by the reaction is equivalent to the amount of glycerol and the nicotinamide adenine dinucleotide can easily be determined quantitatively on the basis of its absorption at 366, 340, or 344 nm.

Generally, a glycerol detection reagent can result in errors due to endogenous enzymes, endogenous substrates and turbidity.

The instant invention overcomes these errors and also provides an extremely fast measurement. This last advantage is particularly important in connection with the fast acting formulation described herein which will hydrolyze the triglycerides to glycerol rapidly and allow rapid measurement of the glycerol content thus obtained.

The invention features a glycerol detection reagent consisting essentially of glycerol kinase, pyruvate kinase, lactate dehydrogenase, adenosine triphosphate, phosphoenol pyruvate, nicotinamide adenosine dinucleotide (reduced form), bovine serum albumen, magnesium sulphate, and a buffer; the ratio of the pyruvate kinase to lactate dehydrogenase is about 1:1; the ratio of pyruvate kinase and lactate dehydrogenase each to glycerol kinase is about 2:1; the magnesium ions are present in the range from 3.1 to 6.0 mM; and the pH of the reagent is in the range of from 6.9 to 7.6.

Preferably, the glycerol kinase, pyruvate kinase, and lactate dehydrogenase are lyophilized and the bovine serum albumen is crystallized and lyophilized.

Preferably, the buffer is in the form of phosphates such as potassium phosphate, monobasic and potassium phosphate, dibasic.

Illustrative non-limiting examples of the practice of the invention are set out below. Numerous other examples can readily be evolved in the light of the guiding principles and teaching contained herein. The examples given herein are intended merely to illustrate the invention and not in any sense to limit the manner in which the invention can be practiced.

Example 1

Tests were carried out to demonstrate the effectiveness of the lipases of the invention to hydrolyze completely within 4 minutes the triglycerides in human serum having a concentration of about 1000 mg triglycerides per dL of serum. This is an unusually high concentration for human serum.

The serum triglycerides were evaluated by conventional alkaline saponification to be 1045±65 mg triglycerides per dL of serum.

Various relative concentrations of *Pseudomonas fluorescens* and *Chromobacterium viscosum* were used as shown in Table 1.

TABLE 1

Lipase units per mL of reaction mixture

| Lipase sample No. | Pseudomonas fluorescens | Chromobacterium viscosum |
|---|---|---|
| 1 | 100 | 0 |
| 2 | 95 | 5 |
| 3 | 90 | 10 |
| 4 | 50 | 50 |
| 5 | 10 | 90 |
| 6 | 0 | 100 |

Each reaction mixture contained the following components:

155.6 $\mu l$ of a lipase sample (containing 540 lipase units per mL in accordance with the relative amounts shown in Table 1),

544.4 $\mu l$ of a 58.5 mM phosphate buffer having a pH of 7.6,

97.9 $\mu l$ deionized water,

2.1 $\mu l$ of a 1.2 M $MgSO_4 \cdot 7H_2O$ solution.

These components were placed in respective tubes and were equilibrated at 30°C. Then, 10 $\mu l$ of serum was added to each tube to complete each reaction mixture and make a total volume of 810 $\mu l$. After 4 minutes, each tube was subjected to rapid heating to about 100°C to quench any reaction which might be taking place.

The tubes were cooled to room temperature and 61.5 $\mu l$ of a 1.2 M $MgSO_4 \cdot 7H_2O$ was added to each tube in order to precipitate any free fatty acids to obtain a clear solution. A clear solution is desirable for measurements made with a spectrophotometer.

Thereafter, a conventional glycerol detection reagent was used in accordance with conventional methods to measure the glycerol content from which the triglycerides concentration can be computed.

The lipase samples determined the triglycerides concentration of the serum as shown in Table 2. Each measured value is a mean of 3 values±standard deviation.

TABLE 2

| Lipase sample number | Lipase ratio Pseudomonas/Chromobacterium | Value of mg/dL of serum |
|---|---|---|
| 1 | 100% Pseudomonas | 1155±148 |
| 2 | 95:5 | 1155±44 |
| 3 | 90:10 | 903±103 |
| 4 | 50:50 | 1333±399 |
| 5 | 10:90 | 1066±148 |
| 6 | 100% Chromobacterium | 1096±74 |

Statistically, the values measured using the Lipase Samples 1 to 6 are equivalent to the value obtained by the prior art alkaline saponification. This shows that the hydrolysis for each Lipase Sample 1 to 6 was an endpoint reaction as well as being faster than the prior art reactions because each reaction was completed within 4 minutes in view of the quenching step.

Example 2

Example 2 was carried out to show that a triglyceride analysis composition according to the invention could obtain an accurate determination rapidly. The instant application discloses the use of at least one bacterial lipase derived from a bacterial source in the group consisting of Pseudomonas species and Chromobacterium species. These lipases alone or in combination with each other have been found to hydrolyze triglycerides rapidly and completely to an endpoint in less than 4 minutes. The preferable lipases are from Pseudomonas fluorescens and Chromobacterium viscosum as well as a combination of these lipases.

Any one of the Lipase Samples 1 to 6 of Example 1 would be suitable for testing herein. The Lipase Sample 3 was utilized having a lipase ratio of Pseudomonas fluorescens to Chromobacterium viscosum of about 90:10 in the amount of 100 lipase units per mL of the composition.

The glycerol detection reagent for the best mode was formulated as follows:

a. All of the components for the reagent were preweighed prior to formulation and stored dessicated at appropriate temperatures.

b. A potassium phosphate buffer was prepared and the pH of the buffer was adjusted to be about 7.5 at room temperature.

c. Bovine serum albumen (crystallized, lyophilized) was added to the buffer.

d. The other components were then added to the buffer as dry powders or as concentrated solutions in the following order:

(i) Adenosine triphosphate (ATP)

(ii) Phosphoenol pyruvate (PEP)

(iii) Nicotinamide adenine dinucleotide (reduced form) (NADH)

(iv) $MgSO_4 \cdot 7H_2O$

(v) Glycerol kinase (lyophilized powder preferred)

(vi) Pyruvate kinase (lyophilized powder preferred)

(vii) Lactate dehydrogenase (lyophilized powder preferred)

(viii) Selected lipase

The completed formulation was used immediately after it was prepared or stored at 4°C for several hours prior to its use.

The amounts of various components used in the reagent are shown in Table 3:

TABLE 3

| Component | Quantity per ml of triglyceride analysis composition |
|---|---|
| ATP | 0.404 mg |
| PEP (monocyclohexyl ammonium salt) | 0.214 mg |
| NADH | 0.223 mg |
| $MgSO_4 \cdot 7H_2O$ | 0.764 mg |
| glycerol kinase | 2.1 IU |
| pyruvate kinase | 4.2 IU |
| lactate dehydrogenase | 4.1 IU |
| potassium phosphate (monobasic) | 1.16 mg |
| potassium phosphate (dibasic) | 10.103 mg |
| Bovine serum albumen | 3.40 mg |

An automated analyzer manufactured and marketed by Union Carbide Corp. under the trademark CentrifiChem 400 System (Analyzer and Pipettor) was used. The instrument settings were such that the following parameters were obtained:

wavelength=340 nm

temperature=30°C

initial reading ($t_o$)=15 seconds

final reading ($t_f$)=4 minutes 15 seconds

time interval=4 minutes

serum volume=5 $\mu$l

water added=50 $\mu$l

triglyceride analyzer composition=350 $\mu$l

The triglycerides concentration of each serum sample was calculated according to the formula:

$$\text{Triglycerides (mg/dL)} = (A_o - A_f) \div \varepsilon_{NAPH}^{340} \times \begin{array}{c} \text{average} \\ \text{molecular} \\ \text{weight} \\ \text{triglyceride} \end{array} \times \begin{array}{c} \text{dilution} \\ \text{factor} \end{array} \times 100$$

$A_o$=initial absorbance ($t_o$=15 seconds)

$A_f$=final absorbance (4 min. 15 sec.)

$\varepsilon_{NAPH}^{340}$ =6.23×10$^3$ abs. units cm$^2$mmol$^{-1}$

dilution factor=80+1=81

Performance characteristics achieved using the CentrifiChem 400® System are shown in Table 4:

### TABLE 4

| | No. of replicates | Average value X̄ (mg/dL) | Std. deviation (mg/dL) | Coefficient of variance % |
|---|---|---|---|---|
| Within-Run Comparison | | | | |
| Normal Control | 22 | 46.6 | 1.87 | 4.0 |
| Elevated Lipid Control | 23 | 141 | 3.0 | 2.0 |
| | | | | |
| Between-Run Comparison | | | | |
| Normal Control | 115 | 49 | — | 3.0 |
| Elevated Lipid Control | 115 | 146 | — | 3.2 |

A comparison of the determination by instant invention to two different prior art methods gave the following correlation statistics:

a) Triglyceride Analysis Reagent vs. SKI method (ultraviolet enzymatic method of SKI Corp.)

No. of Samples=78

$y=1.10x-11$ (least squares line)

$r=0.996$ (correlation coefficient)

b) Triglyceride Analysis Reagent vs. Dow Method (visible enzymatic method of Dow Chemical Corp.)

No. of Samples=56

$y=1.20x-21.0$ (least squares line)

$r=0.974$ (correlation coefficient)

Example 3

Tests were carried out to compare measured triglycerides concentration of human serum for different units of the lipase *Pseudomonas fluorescens*. A pooled serum was used in the procedure of the Example 2. The lipase units were based on the triglyceride analysis composition.

Table 5 shows the results:

### TABLE 5

| Lipase units per mL composition | Triglycerides concentration mg/dL |
|---|---|
| 100 | 248 |
| 125 | 248 |
| 150 | 259 |
| 200 | 260 |

The measured values show consistent values independent of the lipase level over a wide range of lipase units per mL of composition.

Example 4

Tests were carried out to determine the linearity of response of the composition to serum triglycerides concentration as indicated by the relationship between triglycerides concentration and absorbance units. A Centrifichem 400® System was used.

To establish a reference a serum pool was diluted with 0.15 M sodium chloride 1:2 and a conventional method was used to measure triglycerides concentration. The undiluted serum pool was found to contain 1185 mg per dL of serum.

Serum samples containing concentrations of 100, 300, 500, 700, 800, and 1000 mg triglycerides per dL of serum were prepared from aliquots of the serum pool by diluting with 0.15 M sodium chloride.

Using the procedure of the Example 2, the results obtained are shown in Table 6.

## TABLE 6

| Conventional method concentration triglycerides mg/dL | Instant method concentration triglycerides mg/dL | Ratio inst./conv. | Absorbance units |
|---|---|---|---|
| 100 | 106 | 1.06 | 0.102 |
| 300 | 316 | 1.05 | 0.296 |
| 500 | 530 | 1.06 | 0.493 |
| 700 | 751 | 1.07 | 0.697 |
| 800 | 860 | 1.08 | 0.797 |
| 1000 | 1094 | 1.09 | 1.014 |

The instant composition has a surprisingly linear response to the triglycerides concentration according to the relationship shown in Table 6 between concentration and observed absorbance units.

Example 5

Tests were carried out to determine the reaction progress over time for the instant composition.

In accordance with the Example 2, samples of human sera were prepared with the triglycerides concentrations of 80 mg per dL, 140 mg per dL, 230 mg per dL and 350 mg per dL.

Three combinations of lipases were used. The respective *Pseudomonas fluorescens: Chromobacterium viscosum* ratios were 90:10, 75:25, and 50:50. In each case, 100 lipase units per mL of the composition were used.

Using the procedure of the Example 2, it was found that each of the measurements of the human sera for each lipase combination stabilized and was completed well within 4 minutes.

Further tests were carried out to optimize and determine the range of the respective components of the glycerol reagent composition for its use as a single component together with the instant lipase. The tests were run in connection with an automated system as in the Example 2. The following parameters were determined:

A. The ratio of the enzymes glycerol kinase, pyruvate kinase, and lactic dehydrogenase was found to be critical. The ratio of pyruvate kinase to lactic dehydrogenase should be about 1:1 and the ratio of glycerol kinase to either of the other enzymes should be about 1:2. Changes in these ratios affect linearity and sensitivity. A reduction in overall concentration will eventually reduce overall reaction rate.

B. For NADH, the initial absorbance reading at 340 nm determines the linearity. Freshly prepared composition has a theoretical absorbance value=340 nm of 1.8 due to NADH. An absorbance value at 340 nm of 1.0 provides good linearity for triglycerides concentrations up to at least 1000 mg per dL serum.

C. Phosphate was selected as the buffer to minimize interference from serum-containing phosphatases. High levels of the phosphate buffer can tend to inhibit the glycerol detection reagent or produce a magnesium phosphate precipitation. Low levels can be insufficient to provide buffering. Other buffers can be used satisfactorily in accordance with prior art practice.

D. Magnesium ions in the range of from about 3.1 mM to about 6.0 mM has been found acceptable, as tested with magnesium sulfate. The higher levels tended to result in a precipitation in the presence of $NH_4+$.

E. A pH range of from about 6.9 to about 7.6 has been found acceptable within the constraint on the buffer given above. NADH stability tends to decrease for a pH below about 7. Preferably, the glycerol detection reagent should have a pH in the range of from about 7.5 to about 7.6.

## Claims

1. A triglyceride analysis composition for use in the triglyceride determination of serum consisting essentially of at least one bacterial lipase derived from a bacterial source selected from *Pseudomonas fluorescens* and/or *Chromobacterium viscosum* and in the amount of at least 700 lipase units per mg triglycerides in the serum and a glycerol detection reagent consisting essentially of glycerol kinase, pyruvate kinase, lactate dehydrogenase, adenosine triphosphate, phosphoenol pyruvate, nicotinamide adenine dinucleotide (reduced form), bovine serum albumen, magnesium sulphate, and a buffer; the ratio of the pyruvate kinase to lactate dehydrogenase is about 1:1, the ratio of pyruvate kinase and lactate dehydrogenase each to glycerol kinase is about 2:1; the magnesium ions are present in the range from 3.1 to 6.0 mM; and the pH of the reagent is in the range of from 6.9 to 7.6.

2. The triglyceride analysis composition of claim 1 wherein, with regard to said glycerol detection reagent, said glycerol kinase, pyruvate kinase and lactate dehydrogenase are lyophilized, said bovine serum albumen is crystallized and lyophilized and said buffer is selected from the group consisting of phosphates.

3. A method of triglyceride determination of serum comprising the steps of combining the serum with a composition consisting essentially of at least one bacterial lipase derived from a bacterial source

7

selected from *Pseudomonas fluorescens* and/or *Chromobacterium viscosum* and in the amount of at least 700 lipase units per mg triglycerides in the serum and a glycerol detection reagent consisting essentially of glycerol kinase, pyruvate kinase, lactate dehydrogenase, adenosine triphosphate, phosphoenol pyruvate, nicotinamide adenine dinucleotide (reduced form), bovine serum albumen, magnesium sulphate, and a buffer; the ratio of the pyruvate kinase to lactate dehydrogenase is about 1:1, the ratio of pyruvate kinase and lactate dehydrogenase each to glycerol kinase is about 2:1; the magnesium ions are present in the range from 3.1 to 6.0 mM; and the pH of the reagent is in the range of from 6.9 to 7.6 and measuring the glycerol content obtained.

4. The method of triglyceride determination of serum according to claim 3 wherein, with regard to said glycerol detection reagent, said glycerol kinase, pyruvate kinase and lactate dehydrogenase are lyophilized, said bovine serum albumen is crystallized and lyophilized and said buffer is selected from the group consisting of phosphates.

## Patentansprüche

1. Zusammensetzung für die Analyse von Triglyceriden im Serum, bestehend im wesentlichen aus mindestens einer bakteriellen Lipase, die aus einer bakteriellen Quelle stammt aus der Gruppe *Pseudomonas* fluorescens und/oder *Chromobacterium* viscosum und in einer Menge von mindestens 700 Lipase-Einheiten pro mg der Triglyceride im Serum sowie aus einem Glycerin-Nachweisreagens, bestehend im wesentlichen aus Glycerinkinase, Pyruvatkinase, Lactatdehydrogenase, Adenosintriphosphate, Phosphoenolpyruvat, Nikotinamid-Adenin-Dinucleotid (reduzierte Form), Rinderserum-Albumen, Magnesiumsulfat und einem Puffer, wobei das Verhältnis von Pyruvatkinase zu Lactatdehydrogenase etwa 1:1 ist, das jeweilige Verhältnis von Pyruvatkinase und Lactatdehydrogenase zu Glycerinkinase etwa 2:1 ist, die Magnesiumionen in einem Bereich von 3,1 bis 6,0 mM anwesend sind und der pH-Wert des Reagens in einem Bereich von 6,9 bis 7,6 ist.

2. Zusammensetzung für die Analyse von Triglyceriden nach Anspruch 1, dadurch gekennzeichnet, daß bezüglich des Glycerin-Nachweisreagens die Glycerinkinase, Pyruvatkinase und Lactatdehydrogenase lyophilisiert sind, das Rinderserum-Albumen kristallisiert und lyophilisiert ist und der Puffer aus der Gruppe der Phosphate ausgewählt ist.

3. Verfahren zur Bestimmung von Triglyceriden im Serum, gekennzeichnet, durch die Stufen des Kombinierens des Serums mit einer Zusammensetzung, bestehend um wesentlichen aus mindestens einer bakteriellen Lipase, abgeleitet aus einer bakteriellen Quelle aus der Gruppe *Pseudomonas* fluorescens und/oder *Chromobacterium* viscosum und in einer Menge von mindestens 700 Lipase-Einheiten pro mg der Triglyceride im Serum und mit einem Glycerin-Nachweisreagens, bestehdn im wesentlichen aus · Glycerinkinase, Pyruvatkinase, Lactatdehydrogenase, Adenosintriphosphate, Phosphoenolpyruvat, Nikotinamid-Adenin-Dinucleotid (reduzierte Form), Rinderserum-Albumen, Magnesiumsulfat und einem Puffer, wobei das Verhätlnis von Pyruvatkinase zu Lactatdehydrogenase etwa 1:1 ist, das jeweilige Verhältnis von Pyruvatkinase und Lactatdehydrogenase zu Glycerinkinase etwa 2:1 ist, die Magnesiumionen in einem Bereich von 3,1 bis 6,0 mM anwesend sind und der pH-Wert des Reagens in einem Bereich von 6,9 bis 7,6 ist, und Messens des erhaltenen Glyceringehalts.

4. Verfahren zur Bestimmung von Triglyceriden im Serum nach Anspruch 3, dadurch gekennzeichnet, daß bezüglich des Glycerin-Nachweisreagens die Glycerinkinase, Pyruvatkinase und Lactatdehydrogenase lyophilisiert sind, das Rinderserum-Albumen kristallisiert und lyophilisiert ist und der Puffer aus der Gruppe der Phosphate ausgewählt ist.

## Revendications

1. Une composition d'analyse des triglycérides pour l'utilisation dans la détermination des triglycérides du sérum constituée essentiellement d'au moins une lipase bactérienne dérivée d'une source bactérienne choisie parmi *Pseudomonas fluorescens* et/ou *Chromobacterium viscosum* en une quantité d'au moins 700 unités de lipase/mg de triglycérides dans le sérum et d'un réactif de détection du glycérol constitué essentiellement de glycérol-kinase, de pyruvate-kinase, de lacticodéshydrogénase, d'adénosine-triphosphate, de phospho-énol-pyruvate, de nicotinamide-adénine-dinucléotide (forme réduite), de sérum-albumine bovine, de sulfate de magnésium et d'un tampon; le rapport de la pyruvate-kinase à la lacticodéshydrogénase est d'environ 1/1, le rapport de la pyruvate-kinase et de la lacticodéshydrogénase, chacune par rapport à la glycérol-kinase, est d'environ 2/1; les ions magnésium sont présents dans la gamme de 3,1 à 6,0 mM et le pH du réactif est dans la gamme de 6,9 à 7,6.

2. La composition d'analyse des triglycérides de la revendication 1, dans laquelle, en ce qui concerne ledit réactif de détection du glycérol, lesdites glycérol-kinase, pyruvate-kinase et lacticodéshydrogénase sont lyophilisées, ladite sérum-albumine bovine est cristallisée et lyophilisée et ledit tampon est choisi parmi le groupe constitué des phosphates.

3. Un procédé de détermination des triglycérides du sérum comprenant les stades de combinaison du sérum avec une composition constituée essentiellement d'au moins une lipase bactérienne dérivée d'une source bactérienne choisie parmi *Pseudomonas fluorescens* et/ou

# 0 045 032

*Chromobacterium viscosum* en une quantité d'au moins 700 unités de lipase/mg de triglycérides dans le sérum et d'un réactif de détection du glycérol constitué essentiellement de glycérol-kinase, de pyruvate-kinase, de lacticodéshydrogénase, d'adénosine-triphosphate, de phospho-énol-pyruvate, de nicotinamide-adénine-dinucléotide (forme réduite), de sérum-albumine bovine, de sulfate de magnésium et d'un tampon; le rapport de la pyruvate-kinase à la lacticodéshydrogénase est d'environ 1/1, le rapport de la pyruvate-kinase et de la lacticodéshydrogénase, chacune relativement à la glycérol-kinase, est d'environ 2/1, les ions magnésium sont présents dans la gamme de 3,1 à 6,0 mM et le pH du réactif est dans la gamme de 6,9 à 7,6 et la mesure de la teneur en glycérol obtenue.

4. Le procédé de détermination des triglycérides du sérum selon la revendication 3, dans lequel, en ce qui concerne ledit réactif de détection du glycérol, lesdites glycérol-kinase, pyruvate-kinase et lacticodéshydrogénase sont lyophilisées, ladite sérum-albumine bovine est cristallisée et lyophilisée et ledit tampon est choisi parmi le groupe constitué des phosphates.